# EUROPÄISCHE PATENTANMELDUNG

(11) **EP 1 407 797 A1**
(43) Veröffentlichungstag der Anmeldung: **14.04.2004**
(21) Anmeldenummer: 03022360.6
(22) Anmeldetag: 04.10.2003
(51) Int. Cl.: A61M 16/04

(54) **Sprechventilanordnung**

(30) Priorität: 08.10.2002 DE 10246929
(71) Anmelder: Willy Rüsch GmbH, 71394 Kernen-Rommelshausen (DE)
(72) Erfinder: Wurster, Helmut, 75038 Oberderdingen (DE)
(74) Vertreter: KOHLER SCHMID + PARTNER

(57) **Zusammenfassung**

Bei einer Sprechventilanordnung (15) mit einer Ventilklappe (12) und mit einem Ventilgehäuse (1), das eine patientenseitige und eine pätientenabgewandte Öffnung (2, 3) und einen dazwischen liegenden Innenraum (4) aufweist, ist im Innenraum (4) ein lösbares Sprechventil (10) angeordnet, das einen Ventilkörper (11) und die mit dem Ventilkörper (11) schwenkbar verbundene Ventilklappe (12) umfasst, wobei die Ventilklappe (12) an der patientenabgewandten Öffnung (2) angeordnet ist und diese bei Exspiration abdeckt. Diese Anordnung vermeidet beim Ein- und Ausatmen eines Patienten eine verstärkte Geräuschentwicklung am Ventilgehäuse (1) und die Sprechventilanordnung ist einfach zusammenbaubar.

## Beschreibung

### Stand der Technik

Die Erfindung betrifft eine Sprechventilanordnung mit einer Ventilklappe und mit einem Ventilgehäuse, das eine patientenseitige und eine patientenabgewandte Öffnung und einen dazwischen liegenden Innenraum aufweist.

Patienten mit operativ angelegten Öffnungen der Trachea unterhalb des Schildknorpels können ohne Hilfsmittel nicht mehr sprechen. Der Grund hierfür ist, dass die Ein- und Ausatmung durch eine in die Trachea eingeführte Tracheostomiekanüle erfolgt. Die Ausatmungsluft strömt nicht mehr durch den Kehlkopf und kann die Stimmbänder nicht zum Schwingen bringen. Deshalb werden Sprechventile eingesetzt, die den Luftstrom bei Inspiration durch die Ventilöffnung treten lassen und das Ventil bei Exspiration verschließen, so dass Luft durch Öffnungen in der Tracheostomiekanüle und/oder entlang des äußeren Umfanges der Kanüle entlang in Richtung Kehlkopf entweichen kann und dort die Stimmbänder zum Schwingen bringt.

Werden als Sprechventil Ventilklappen aus harten Materialien verwendet, ist beim Sprechen ein störendes, klickendes Geräusch zu hören. Die Montage dieser Ventile erweist sich oft als schwierig, ebenso die Reinigung.

### Aufgabe der Erfindung

Aufgabe der vorliegenden Erfindung ist es deshalb, eine Sprechventilanordnung zu schaffen, die einen möglichst geringen Strömungswiderstand hat, die einfach zusammengebaut werden kann und bei der ein wahrnehmbares Geräusch beim Sprechen weitestgehend vermieden wird.

### Gegenstand der Erfindung

Erfindungsgemäß wird diese Aufgabe dadurch gelöst, dass im Innenraum ein lösbares Sprechventil angeordnet ist, das einen Ventilkörper und die mit dem Ventilkörper schwenkbar verbundene Ventilklappe umfasst, wobei die Ventilklappe an der patientenabgewandten Öffnung angeordnet ist und diese bei Exspiration abdeckt.

Dadurch, dass das Sprechventil lösbar im Ventilgehäuse angeordnet ist, kann die Sprechventilanordnung leicht zerlegt und wieder zusammengebaut werden. Dies ermöglicht eine einfache Reinigung der gesammten Sprechventilanordnung bzw. des Sprechventils und des Ventilgehäuses, sowie bei Bedarf den Austausch des Sprechventils.

Bei einer besonders bevorzugten Ausführungsform sind der Ventilkörper und die Ventilklappe materialschlüssig miteinander verbunden.

Die materialschlüssige Verbindung wird vorzugsweise durch einen Materialsteg realisiert. Dadurch kann das Sprechventil, bestehend aus Ventilkörper und Ventilklappe, in einem einzigen Spritzgussteil gefertigt werden. Die gesamte Sprechventilanordnung kann dadurch besonders kostengünstig hergestellt werden. Das Sprechventil und das Ventilgehäuse werden jeweils in einem einzigen Schrittgussvorgang gefertigt.

Ist der Ventilkörper ringförmig ausgebildet, so kann er sich besonders gut an den Innenraum des Ventilgehäuses anpassen. Ist der Ventilkörper zudem zylindrisch ausgebildet, dann kann ein Kippen des Ventilkörpers bei der Montage im Ventilgehäuse vermieden werden. Unter ringförmig im Sinne der Erfindung wird nicht nur kreisringförmig verstanden, sondern jede eine Durchgangsöffnung begrenzende Form. Dabei ist es denkbar, dass eine im Wesentlichen kreisrunde Durchgangsöffnung in einem quadratischen oder rechteckigen Körper ausgebildet ist.

Bei einer weiteren Ausführungsform ist die Ventilklappe im Lumen des Ventilgehäuses angeordnet und entspricht etwa der Querschnittsform des Lumens.

Durch diese Maßnahme kann die Ventilklappe in Längsrichtung des Lumens ungestört dem Luftstrom ausweichen, wenn der Patient einatmet.

Bei einer bevorzugten Weiterbildung sind das Lumen und die Ventilklappe kreisrund ausgebildet.

Diese Maßnahme verhindert ein Verhaken und Verkanten der Ventilklappe im Ventilkörper, wenn die Ventilklappe aufgrund des Luftstroms bei Inspiration verschwenkt wird. Außerdem kann die Ventilklappe bei Exspiration ungestört in ihre Ausgangsposition zurück schwenken. Die Funktionssicherheit der Sprechventilanordnung wird daher erhöht.

Vorteilhafterweise sind der Ventilkörper und die Ventilklappe aus elastischem Weichkunststoff, insbesondere aus Silikon, hergestellt.

Dies ermöglicht eine besonders einfache Montage der Sprechventilanordnung, da das Sprechventil für die Montage verformt werden kann und durch die patientenabgewandte Öffnung des Ventilgehäuses in das Ventilgehäuse eingeschoben werden kann. Im Ventilgehäuse nimmt das Sprechventil wieder seine ursprüngliche Form an und schmiegt sich im Innenraum von innen in seiner Ursprungsform deformationsfrei gegen das Ventilgehäuse. Eine Ventilklappe aus Silikon vermeidet ein störendes Geräusch beim Sprechen mit der Sprechventilanordnung. Außerdem setzt sie der Atemströmung nur einen geringen Strömungswiderstand entgegen.

Wenn die patientenabgewandte Öffnung des Ventilgehäuses kleiner ist als die Ventilklappe, ist ein gutes Abdichten der patientenabgewandten Öffnung durch die Ventilklappe sichergestellt. Das die patientenabgewandte Öffnung begrenzende Material des Ventilgehäuses bildet einen Anschlag für die Ventilklappe.

Bei einer bevorzugten Ausführungsform ist im Innenraum des Ventilgehäuses ein Anschlag für den Ventilkörper vorgesehen.

Durch diese Maßnahme wird ein Verrutschen des Ventilkörpers im Ventilgehäuse verhindert. Außerdem kann der Ventilkörper besonders einfach im.Ventilgehäuse montiert werden.

Vorzugsweise ist der Ventilkörper in einer umlaufenden Nut des Ventilgehäuses gelagert.

Durch die umlaufende Nut wird die Lage des Ventilkörpers im Ventilgehäuse exakt bestimmt. Ein Kippen und Verrutschen des Ventilkörpers im Ventilgehäuse wird dadurch ausgeschlossen.

Weitere Merkmale und Vorteile der Erfindung ergeben sich aus der nachfolgenden Beschreibung eines Ausführungsbeispiels der Erfindung, anhand der Figuren der Zeichnung, die erfindungswesentliche Einzelheiten zeigen, und aus den Ansprüchen. Die einzelnen Merkmale können je einzeln für sich oder zu mehreren in beliebiger Kombination bei einer Variante der Erfindung verwirklicht sein.

### Zeichnung

Ein Ausführungsbeispiel der erfindungsgemäßen Sprechventilanordnung ist in der schematischen Zeichnung dargestellt und wird in der nachfolgenden Beschreibung erläutert. Es zeigt:
- Fig. 1: einen Längsschnitt durch ein Ventilgehäuse;
- Fig. 2: einen Längsschnitt durch ein Sprechventil;
- Fig. 3: einen Längsschnitt durch eine Sprechventilanordnung im zusammengebauten Zustand; und
- Fig. 4: eine Draufsicht auf das Sprechventil.

### Beschreibung des Ausführungsbeispiels

Die Figuren sind sehr schematisch dargestellt, um die wesentlichen erfinderischen Merkmale zu verdeutlichen. In den Darstellungen sind die Dimensionen nur beispielhaft und nicht maßstäblich zu verstehen.

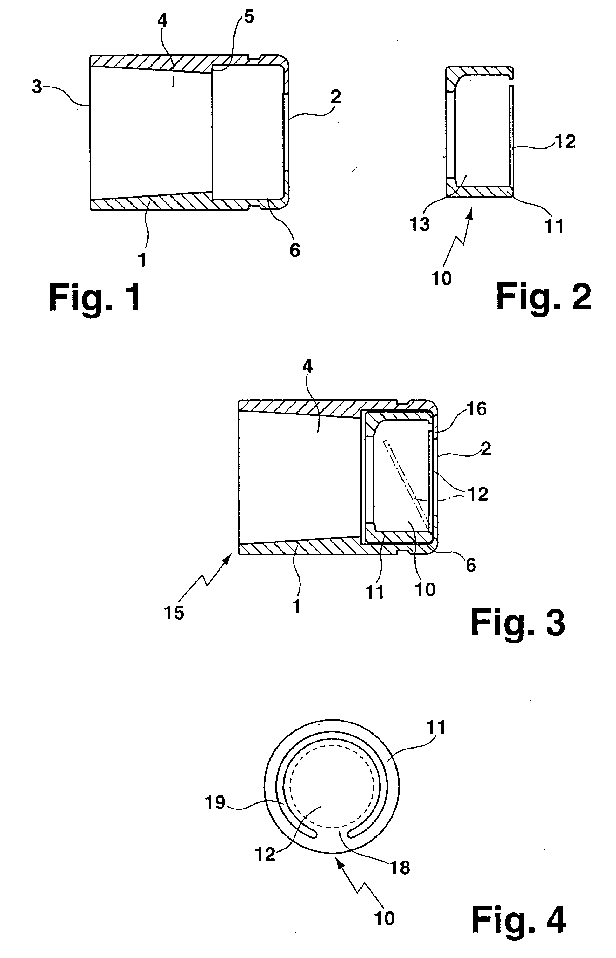

**Fig. 1** zeigt einen Längsschnitt durch ein Ventilgehäuse **1** einer Sprechventilanordnung. Das Ventilgehäuse 1 weist eine patientenabgewandte Öffnung **2** und eine patientenseitige Öffnung **3** mit dazwischen liegendem Innenraum **4** auf. Im Innenraum 4 ist ein Anschlag **5** vorgesehen, der Teil einer umlaufenden Ringnut **6** ist. In die patientenseitige Öffnung 3 kann ein Konnektor (z.B. 15-mm Normkonnektor) einer beliebigen Tracheostomiekanüle eingeschoben werden, welcher im sich zur patientenseitigen Öffnung 3 konisch erweiternden Lumen des Ventilgehäuses 1 formschlüssig gehalten ist.

In der **Fig. 2** ist ein Sprechventil **10** gezeigt, das einen Ventilkörper **11** und eine damit schwenkbar verbundene Ventilklappe **12** umfasst. Die Ventilklappe 12 ist mit dem Ventilkörper 11 durch einen Materialsteg verbunden. Die Ventilklappe 12 kann ungehindert in das Lumen **13** des Ventilkörpers 11 ausweichen, wenn der Patient einatmet.
Das Sprechventil 10 ist in der Ringnut 6 des Ventilgehäuses 1 der Fig. 1 gelagert.

In der **Fig. 3** ist ein Längsschnitt der zusammengebauten Sprechventilanordnung **15** gezeigt. Der Ventilkörper 11 ist an die Ringnut 6 des Ventilgehäuses 1 angepasst. Das Ventilgehäuse 1 ist ein Spritzgussteil, welches aus hartem Kunststoff gefertigt ist. Das Sprechventil 10 ist aus einem elastischen Weichkunststoff ebenfalls als Spritzgussteil gefertigt. Zum Zusammenbau der Sprechventilanorndnung 15 wird das Sprechventil 10 zusammengepresst und durch die patientenabgewandte Öffnung 2 geschoben. Sobald das Sprechventil 10 sich im Innenraum 4 des Ventilgehäuses befindet, dehnt es sich wieder selbsttätig aus und füllt den Raum der Ringnut 6 aus. Mit durchgezogenen Linien ist die Ventilklappe 12 in der Position gezeigt, wenn der Patient spricht. Dabei liegt die Ventilklappe 12 an dem die patientenabgewandte Öffnung 2 begrenzenden Material 16 an. Es kann keine Luft durch das Sprechventil 10 entweichen. Mit gestrichelten Linien ist die Ventilklappe 12 gezeigt, wenn der Patient einatmet. Die Ventilklappe 12 ist verschwenkt und gibt die patientenabgewandte Öffnung 2 frei, sodass Luft durch die Sprechventilanordnung 15 zur Lunge des Patienten strömen kann.

**Fig. 4** zeigt eine Draufsicht auf das Sprechventil 10. Die Ventilklappe 12 ist mit dem Ventilkörper 11 über einen formstabilen, elastischen Materialsteg 18 verbunden. Der Querschnitt der Ventilklappe 12 ist im wesentlichen kreisrund ausgebildet und weist damit in etwa dieselbe Querschnittsform wie das Lumen des Ventilkörpers 11 auf. Die Ventilklappe 12 ist im Lumen des Ventilkörpers 11 angeordnet und abgesehen vom Materialsteg **18** vom Ventilkörper 11 durch einen Spalt **19** beabstandet. Bei Druck auf die Ventilklappe 12 in Zeichenebene weicht die Ven tilklappe 12 in das Lumen des Ventilkörpers 11 aus. Die zwischen dem Rand und der kreisrund gezeichneten unterbrochenen Linienführung ausgebildete Kreisringfläche der Ventilklappe 12 ist als Auflagefläche für eine Gegenfläche am Ventilgehäuse 1 ausgebildet, wobei die Auflagefläche an der Gegenfläche anliegt, wenn der Ventilkörper 11 das Lumen des Ventilgehäuses verschließt.

Bei einer Sprechventilanordnung 15 mit einer Ventilklappe 12 und mit einem Ventilgehäuse 1, das eine patientenseitige und eine patientenabgewandte Öffnung 2, 3 und einen dazwischen liegenden Innenraum 4 aufweist, ist im Innenraum 4 ein lösbares Sprechventil 10 angeordnet, das einen Ventilkörper 11 und die mit dem Ventilkörper 11 schwenkbar verbundene Ventilklappe 12 umfasst, wobei die Ventilklappe 12 an der patientenabgewandten Öffnung 2 angeordnet ist und diese bei Exspiration abdeckt. Diese Anordnung gewährleistet einen niedrigen Strömungswiderstand, vermeidet beim Ein- und Ausatmen eine störende Geräuschentwicklung am Ventilgehäuse 1 und die Sprechventilanordnung ist einfach zusammenzubauen und zu reinigen.

## Patentansprüche

1. Sprechventilanordnung (15) mit einer Ventilklappe (12) und mit einem Ventilgehäuse (1), das eine patientenseitige und eine patientenabgewandte Öffnung (2, 3) und einen dazwischen liegenden .Innenraum (4) aufweist, **dadurch gekennzeichnet, dass** im Innenraum (4) ein lösbares Sprechventil (10) angeordnet ist, das einen Ventilkörper (11) und die mit dem Ventilkörper (11) schwenkbar verbundene Ventilklappe (12) umfasst, wobei die Ventilklappe (12) an der patientenabgewandten Öffnung (2) angeordnet ist und diese bei Exspiration abdeckt.

2. Sprechventilanordnung nach Anspruch 1, **dadurch gekennzeichnet, dass** der Ventilkörper (11) und die Ventilklappe (12) materialschlüssig miteinander verbunden sind.

3. Sprechventilanordnung nach einem der vorhergehenden Ansprüche, **dadurch gekennzeichnet, dass** der Ventilkörper (11) ringförmig ausgebildet ist.

4. Sprechventilanordnung nach einem der vorhergehenden Ansprüche, **dadurch gekennzeichnet, dass** die Ventilklappe (12) im Lumen (13) des Ventilkörpers (11) angeordnet ist und etwa der Querschnittsform des Lumens (13) entspricht.

5. Sprechventilanordnung nach Anspruch 4, **dadurch gekennzeichnet, dass** das Lumen (13) und die Ventilklappe (12) kreisrund ausgebildet sind.

6. Sprechventilanordnung nach einem der vorhergehenden Ansprüche, **dadurch gekennzeichnet, dass** der Ventilkörper (11) und die Ventilklappe (12) aus elastischem Weichkunststoff, insbesondere Silikon, hergestellt sind.

7. Sprechventilanordnung nach einem der vorhergehenden Ansprüche, **dadurch gekennzeichnet, dass** die patientenabgewandte Öffnung (2) des Ventilgehäuses (1) kleiner ist als die Ventilklappe (12).

8. Sprechventilanordnung, **dadurch gekennzeichnet, dass** im Innenraum (4) des Ventilgehäuses (1) ein Anschlag (5) für den Ventilkörper (11) vorgesehen ist.

9. Sprechventilanordnung, **dadurch gekennzeichnet, dass** der Ventilkörper (11) in einer umlaufenden Nut (6) des Ventilgehäuses (1) gelagert ist.
